# EUROPEAN PATENT SPECIFICATION

(11) **EP 1 412 481 B1**
(45) Date of publication and mention of the grant of the patent: **01.04.2015**
(21) Application number: 02756367.5
(22) Date of filing: 03.07.2002
(51) Int. Cl.: C12N 5/077, C12N 5/0775

(54) **MESENCHYMAL CELLS AND OSTEOBLASTS FROM HUMAN EMBRYONIC STEM CELL**
MESENCHYMZELLEN UND OSTEOBLASTEN AUS MENSCHLICHER EMBRYONALER STAMMZELLE
OSTEOBLASTES ET CELLULES MESENCHYMATEUSES PROVENANT DE CELLULE SOUCHE EMBRYONNAIRE HUMAINE

(30) Priority: 06.07.2001 US 303732 P
(43) Date of publication of application: 28.04.2004
(73) Proprietor: Asterias Biotherapeutics, Inc., Menlo Park, CA 94025 (US)
(72) Inventor: XU, Chunhui, Cupertino, CA 95014 (US); THIES, R. Scott, Pleasanton, CA 94566 (US)
(74) Representative: Bassil, Nicholas Charles
(86) International application number: PCT/US2002/020998
(87) International publication number: WO 2003/004605

(56) References cited:
- US-A- 5 972 703
- US-A- 6 077 987
- ITSKOVITZ-ELDOR J ET AL: "DIFFERENTIATION OF HUMAN EMBRYONIC STEM CELLS INTO EMBYOID BODIES COMPRISING THE THREE EMBRYONIC GERM LAYERS" MOLECULAR MEDICINE, BLACKWELL SCIENCE, CAMBRIDGE, MA, US, vol. 6, February 2000 (2000-02), pages 88-95, XP000942869 ISSN: 1076-1551
- BUTTERY L D K ET AL: "Differentiation of osteoblasts and in vitro bone formation from murine embryonicstem cells" TISSUE ENGINEERING, LARCHMONT, NY, US, vol. 7, no. 1, February 2001 (2001-02), pages 89-99, XP002978343 ISSN: 1076-3279
- GEPSTEIN L: "Derivation and potential applications of human embryonic stem cells" CIRCULATION RESEARCH, GRUNE AND STRATTON, BALTIMORE, US, vol. 91, no. 10, 15 November 2002 (2002-11-15), pages 866-876, XP002267407 ISSN: 0009-7330
- MAYR-WOHLFART U ET AL: "BMP-4 of Xenopus laevis stimulates differentiation of human primary osteoblast-like cells.", THE JOURNAL OF BONE AND JOINT SURGERY. BRITISH VOLUME JAN 2001 LNKD- PUBMED:11245525, vol. 83, no. 1, January 2001 (2001-01), pages 144-147, ISSN: 0301-620X
- CHUNG YOUNG ET AL: "Human embryonic stem cell lines generated without embryo destruction", CELL STEM CELL, CELL PRESS, US, vol. 2, no. 2, 7 February 2008 (2008-02-07), pages 113-117, XP002604696, ISSN: 1934-5909, DOI: 10.1016/J.STEM.2007.12.013
- KLIMANSKAYA I ET AL: "HUMAN EMBRYONIC STEM CELL LINES DERIVED FROM SINGLE BLASTOMERES", NATURE: INTERNATIONAL WEEKLY JOURNAL OF SCIENCE, NATURE PUBLISHING GROUP, UNITED KINGDOM, vol. 444, no. 7118, 1 November 2006 (2006-11-01), pages 481-485, XP009076989, ISSN: 0028-0836, DOI: 10.1038/NATURE05142

## Description

### TECHNICAL FIELD

This invention relates to a culture medium and a growth environment for obtaining osteoprogenitors and/or osteoblasts, using special culture conditions and selection techniques.

### BACKGROUND

Regenerative medicine is an important new initiative in the biotechnology industry. Methods are being developed to produce cultures of specialized cells that are proposed for use in promoting tissue repair and the healing of diseases for which previous therapeutic regimens are unsatisfactory.

One area of emerging interest is the use of cultured cells to enhance or repair bony tissues. There have been several published reports of osteoblast progenitor cells and mesenchymal stem cells under development.

U.S. Patents 5,691,175, 5,681,701, and 5,693,511 (Mayo Foundation) describe immortalized normal human fetal osteoblastic cells that express a temperature-sensitive mutant of simian virus 40 large T antigen. U.S. Patent 5,972,703 (Michigan) reports compositions of bone precursor cells that are not hematopoietic and which can differentiate into osteoblasts upon exposure to a bone growth factor, depositing calcium into the extracellular matrix. U.S. Patent 6,200,602 (DuPuy Orthopaedics) reports the isolation of cartilage or bone precursor cells from hematopoietic and non-hematopoietic cells, and proposes their use in bone and cartilage regeneration.

International patent publication WO 95/22611 (Michigan) reports methods for transferring nucleic acids into bone cells in situ for stimulating bone progenitor cells. Type II collagen and osteotropic genes are explored for use in promoting bone growth, repair and regeneration in an animal model. International patent publication WO 99/39724 (Oregon) proposes to treat bony defects with osteoblast progenitor cells. The cells may be transformed to express a bone morphogenetic protein such as BMP-2.

The subject of bone stem cells is reviewed by J.E. Aubin (J. Cell Biochem. Suppl. 30/31:73, 1998). Stem and primitive osteoprogenitors and related mesenchymal precursors contribute to replacement of osteoblasts in bone turnover and in fracture healing. The article puts forward the hypothesis that the mature osteoblast phenotype is heterogeneous with subpopulations of osteoblasts expressing subsets of the known osteoblast markers, raising the possibility of multiple parallel differentiation pathways and different progenitor pools.

Joyner et al. (Bone 21:1, 1997) report the identification and enrichment of human osteoprogenitor cells using differentiation stage-specific monoclonal antibody. A particular antibody was selected for its reactivity with marrow cultures and immunohistochemical localization in fetal tissues in progenitor cell regions adjacent to osteoblastic cells. In immunopanning, the antibody selected stromal fibroblastic colony forming units (CFU-F). Thies et al. (Endocrinology 130:1318, 1992) report that bone morphogenic protein-2 induces osteoblastic differentiation in a line of stromal cells, increasing alkaline phosphatase activity in a dose-dependent manner without affecting cell proliferation.

Liechty et al. (Nature Med. 6:1282, 2000) reported that human mesenchymal stem cells (MSC) engraft and demonstrate site-specific differentiation after in utero transplantation in sheep. An MSC population was obtained by iliac crest aspiration from normal human donors, and transplanted into sheep before the development of immunological competence. The cells engrafted and persisted in multiple tissues for 13 months. The article reports that they underwent site-specific differentiation into chondrocytes, adipocytes, myocytes, cardiomyocytes, bone marrow stromal cells and thymic stroma.

U.S. Patent 5,908,784 (Case Western Reserve) is related to obtaining human MSCs by taking bone marrow cells, growing them in BGJ*_{b}* medium with fetal calf serum, and identifying them using monoclonal antibody. Induction of chondrocytes in vitro involves contacting a packed cell pellet with a chondroinductive agent. U.S. Patent 5,486,359 (Osiris Therapeutics) reports isolation of human mesenchymal stem cells that can differentiate into bone, cartilage, muscle, or marrow stroma. International patent publication WO 97/40137 (Osiris) proposes a system for regeneration and augmentation of bone using mesenchymal stem cells. Compositions comprise MSCs or fresh bone marrow cells combined with a ceramic material or resorbable biopolymer.

It is unclear whether any of the cell preparations exemplified in these publications can be produced in sufficient quantities for mass marketing as a therapeutic composition for bone repair.

### Undifferentiated pluripotent stem cells of embryo origin

A different area of medical research deals with stem cells that have not committed to producing progeny of any particular lineage. A number of recent discoveries have raised expectations that embryonic cell lines may become a source for cells and tissues useful in regenerative medicine for a wide variety of degenerative conditions. Embryonic stem cells are described as pluripotent, because they are considered capable of differentiating into a variety of cell types (R.A. Pedersen, Scientif. Am. 280(4):68, 1999).

Early work on embryonic stem cells was done using inbred mouse strains as a model (reviewed in Robertson, Meth. Cell Biol. 75:173, 1997; and Pedersen, Reprod. Fertil. Dev. 6:543,1994). However, compared with mouse ES cells, monkey and human pluripotent cells have proven to be much more fragile, and do not respond to the same culture conditions. Factors that affect their persistence in culture and their subsequent differentiation are considerably different. Only recently have discoveries been made that allow primate embryonic cells to be cultured ex vivo.

Thomson et al. (Proc. Natl. Acad. Sci. USA 92:7844, 1995) were the first to successfully culture embryonic stem cells from primates, using rhesus monkeys and marmosets as a model. They subsequently derived human embryonic stem (hES) cell lines from human blastocysts (Science 282:114, 1998), coculturing them with mouse embryonic fibroblasts to support their maintenance and growth. Gearhart and coworkers derived human embryonic germ (hEG) cell lines from fetal gonadal tissue (Shamblott et al., Proc. Natl. Acad. Sci. USA 95:13726, 1998), also supported on feeder cells. Both hES and hEG cells have the long-sought characteristics of pluripotent stem cells: they are capable of ongoing proliferation in vitro without differentiating, they retain a normal karyotype, and they retain the capacity to differentiate to produce a variety of adult cell types.

Geron Corporation has developed novel tissue culture environments that allow for continuous proliferation of pluripotent stem cells in an environment essentially free of feeder cells. See Australian patent AU 729377, and International Patent Publication WO 01/51616. Being able to culture stem cells in a feeder-free environment provides a system in which cellular compositions can be readily produced that are in compliance with the regulatory requirements for human therapy.

In order to realize the potential of pluripotent stem cells in the management of human health and disease, it is now necessary to develop new paradigms to drive these cells into populations of therapeutically important tissue types.

### SUMMARY

According to the first aspect of the invention there is provided a culture medium for obtaining osteoprogenitors and/or osteoblasts from human embryonic stem (hES) cells or their progeny, comprising BMP 4, dexamethasone, and ascorbic acid or an analog thereof. The culture medium may further comprise beta-glycerophosphate.

This invention provides a growth environment or culture medium for efficient production of osteoprogenitor and/or osteoblast cells that have differentiated from human embryonic stem (hES) cells.

An isolated cell or cell population that proliferates in an in vitro culture may be obtained by differentiating human embryonic stem (hES) cells in said culture medium. Cell populations can comprise at least ~10%, ~30%, or ~60% mesenchymal cells of various types, having characteristics listed elsewhere in this disclosure. For example, osteoblasts and their precursors may express osteocalcin, type 1 collagen and alkaline phosphatase. Mature osteoblasts may express osteocalcin, and be capable of forming an extracellular matrix comprising calcium.

These cells are derived from a human embryonic stem (hES) cell line, thereby sharing the same genome as the line from which they were derived, with any induced genetic alterations. If desired, the cells can be genetically altered to increase proliferative capacity: for example, with an expression vector for telomerase reverse transcriptase. The cells can also be genetically altered to express a bone morphogenic protein.

According to the second aspect of the invention there is provided a growth environment comprising a culture medium according to the first aspect of the invention and a supporting structure.

These and other embodiments of the invention will be apparent from the description that follows.

### DRAWINGS

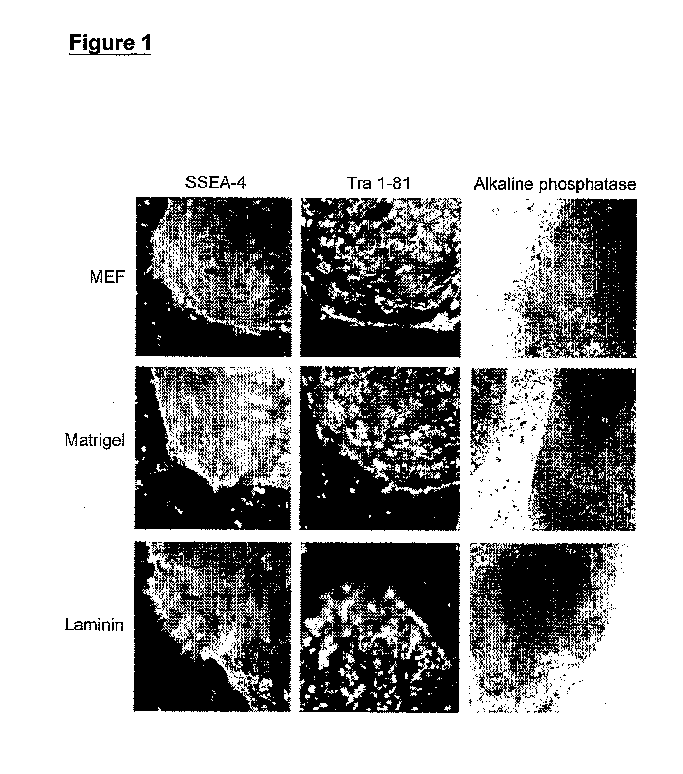

**Figure 1** is a reproduction of micrographs showing marker expression detected by immunocytochemistry for undifferentiated human embryonic stem (hES) cells. The cultures were grown according to conventional methods on mouse embryonic feeder cells, or in a feeder-free environment comprising extracellular matrices Matrigel® or laminin in conditioned medium. hES cells grown in feeder-free culture have phenotypic markers similar to those of hES grown on a feeder layer of primary mouse fibroblasts.

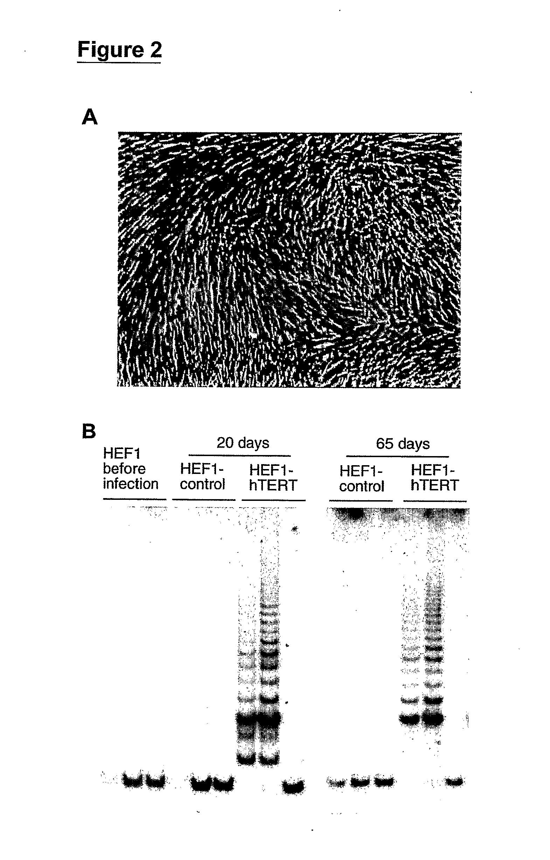

**Figure 2** shows features of a human cell line designated HEF1 that was differentiated from hES cells. Panel A is a copy of a phase contrast micrograph, showing that the HEF1 cell line has morphological characteristics of fibroblasts. Panel B (below) is a copy of the results of a TRAP assay, showing that HEF1 cells transduced with a retroviral vector for telomerase reverse transcriptase (hTERT) acquired telomerase activity.

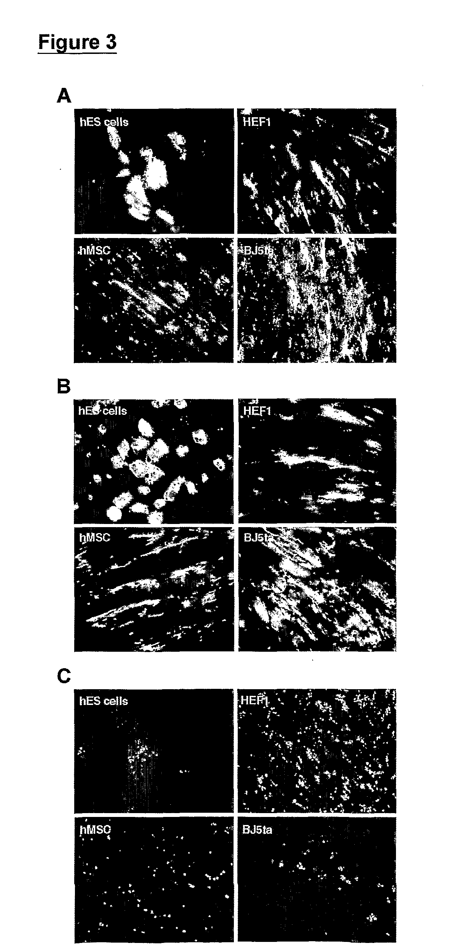

**Figure 3** is a reproduction of micrographs showing marker expression of cell lines that had been subjected to a differentiation paradigm to generate osteoprecursor cells and osteoblasts. The culture medium was replaced with osteoblast induction medium (OIM), and then differentiated for 11 days. The OIM was prepared from a mesenchymal stem cell growth medium, and supplemented with 0.1 µM dexamethasone, 5 µM ascorbic acid-2-phosphate, 10 mM β-glycerophosphate, and 100 ng/mL BMP-4. Cells used were the hES cell line H1, the telomerized hES-derived differentiated cell line HEF1, human mesenchymal stem cells, and BJ5ta fibroblasts.

Panels A and B show immunocytochemistry for the markers osteocalcin and collagen-1. Panel C shows staining for alkaline phosphatase activity. These features are characteristic of cells of the osteoblast lineage, and indicate that both hES cells and HEF1 cells generate osteoblasts when subjected to an appropriate differentiation protocol in vitro.

### DETAILED DESCRIPTION

This invention provides technology that can be used for obtaining osteoprogenitors and/or osteoblasts involved in the turnover and reparation of bone.

If pPS cells are allowed to differentiate in an undirected fashion, a heterogeneous population of cells is obtained expressing markers for a plurality of different tissue types (WO 01/51616; Shamblott et al., Proc. Natl. Acad. Sci. U.S.A. 98:113, 2001 A significant challenge in using pPS cells for therapeutic purposes, or for studying particular cell types in vitro, is to obtain cell populations that comprise a substantial subpopulation that is relatively uniform in characteristics. None of the articles reviewed in the background section of this disclosure teach or suggest a method for deriving osteoblasts or their precursors from embryonic stem cells of any species.

It has now been discovered that substantially homogenous populations of cells of the mesenchymal lineage can be obtained by culturing pluripotent embryonic cells in conditions optimized for cells of this type. Reference Example 2 (below) describes how human embryonic stem (hES) cells can be differentiated into a line of early mesodermal cells. The hES cells were caused to form embryoid bodies, which were then plated under conditions suitable to select a line of cells bearing phenotypic characteristics of mesodermal cells. The isolated cell line was then transduced with telomerase reverse transcriptase, to increase proliferation capacity. This cell line has the capability of self renewal, and of forming progeny of various mature mesenchymal cell types.

In Reference Example 3, the mesenchymal cell line in turn was caused to differentiate into cells of the osteoblast lineage, identified by staining for collagen-1 osteocalcin, and alkaline phosphatase activity. Reference Example 3 also describes how cells having osteoblast characteristics can also be obtained directly by culturing human embryonic stem (hES) cells in an appropriate culture environment. Specifically, the cells were cultured for 11 days in a commercially available mesenchymal cell growth medium, supplemented with 0.1 µM dexamethasone, 5 µM ascorbic acid-2- phosphate, 10 mM β-glycerophosphate, and 100 ng/mL BMP-4.

Certain cell populations obtained using a culture medium or growth environment of this invention contain a high proportion of osteoblasts and their precursors. It is not known whether the culture conditions induce hES cells to adopt an osteoblast phenotype, whether they promote outgrowth of cells of this type, or if they inhibit growth of other cell types - indeed, it is quite possible that several of these mechanisms work in concert to enrich for cells of the desired type. Of course, the mechanism responsible for causing enrichment for cells of the osteoblast lineage is of interest, but it is not necessary to understand the mechanism in order to practice the invention.

The remarkable uniformity and functional properties of the cells produced according to this system make them valuable for developing new therapeutic modalities and as a tool for studying mesenchymal tissues in vitro.

### Definitions

Prototype "primate Pluripotent Stem cells" (pPS cells) are pluripotent cells derived from any kind of embryonic tissue (fetal or pre-fetal tissue), and have the characteristic of being capable under appropriate conditions of producing progeny of different cell types that are derivatives of all of the 3 germinal layers (endoderm, mesoderm, and ectoderm), according to a standard art-accepted test, such as the ability to form a teratoma in 8-12 week old SCID mice, or the ability to form identifiable cells of all three germ layers in tissue culture.

Included in the definition of pPS cells are embryonic cells of various types, exemplified by human embryonic stem (hES) cells, described by Thomson et al. (Science 282:1145, 1998); embryonic stem cells from other primates, such as Rhesus stem cells (Thomson et al., Proc. Natl. Acad. Sci. USA 92:7844, 1995), marmoset stem cells (Thomson et al., Biol. Reprod. 55:254, 1996) and human embryonic germ (hEG) cells (Shamblott et al., Proc. Natl. Acad. Sci. USA 95:13726, 1998). Other types of pluripotent cells are also included in the term. Any cells of primate origin that are capable of producing progeny that are derivatives of all three germinal layers are included, regardless of whether they were derived from embryonic tissue, fetal tissue, or other sources. The pPS cells are not derived from a malignant source. It is desirable (but not always necessary) that the cells be karyotypically normal.

pPS cell cultures are described as "undifferentiated" when a substantial proportion of stem cells and their derivatives in the population display morphological characteristics of undifferentiated cells, clearly distinguishing them from differentiated cells of embryo or adult origin. Undifferentiated pPS cells are easily recognized by those skilled in the art, and typically appear in the two dimensions of a microscopic view in colonies of cells with high nuclear/cytoplasmic ratios and prominent nucleoli. It is understood that colonies of undifferentiated cells within the population will often be surrounded by neighboring cells that are differentiated.

For the purposes of this disclosure, a "mesenchymal cell" can be either a terminally differentiated cell or a proliferative precursor cell committed to form cells of a mesenchymal tissue, such as bone, dental tissue, cartilage, tendon, bone marrow stroma, the hematopoietic lineage, or muscle. Mesenchymal stem cells are included in the term, as are terminally differentiated (post-mitotic) cells and more committed replication-competent cells, such as osteoblast precursor cells. Mesenchymal cells all have the property that they are either terminally differentiated in the mesenchymal lineage, or are restricted to form progeny of the mesenchymal lineage or their precursors. They do not form endodermal or ectodermal cells unless subject to nuclear transfer or otherwise reprogrammed.

In the context of cell ontogeny, the adjective "differentiated" is a relative term. A "differentiated cell" is a cell that has progressed further down the developmental pathway than the cell it is being compared with. Thus, pluripotent embryonic stem cells can differentiate to lineage-restricted precursor cells (such as a mesenchymal stem cell), which in turn can differentiate into other types of precursor cells further down the pathway (such as an osteoblast precursor), and then to an end-stage differentiated cell, which plays a characteristic role in a certain tissue type, and may or may not retain the capacity to proliferate further.

A "differentiation agent", as used in this disclosure, refers to one of a collection of compounds that are used in culture systems of this invention to produce differentiated cells of the mesenchymal lineage (including precursor cells and terminally differentiated cells). No limitation is intended as to the mode of action of the compound. For example, the agent may assist the differentiation process by inducing or assisting a change in phenotype, promoting growth of cells with a particular phenotype or retarding the growth of others, or acting in concert with other agents through unknown mechanisms.

Unless explicitly indicated otherwise, the techniques described herein can be brought to bear without restriction on any type of progenitor cell capable of differentiating into bone.

"Feeder cells" or "feeders" are terms used to describe cells of one type that are co-cultured with cells of another type, to provide an environment in which the cells of the second type can grow. pPS cell populations are said to be "essentially free" of feeder cells if the cells have been grown through at least one round after splitting in which fresh feeder cells are not added to support the growth of the pPS.

A "growth environment" is an environment in which cells of interest will proliferate, differentiate, or mature in vitro. Features of the environment include the medium in which the cells are cultured, any growth factors or differentiation-inducing factors that may be present, and a supporting structure (such as a substrate on a solid surface) if present.

A cell is said to be "genetically altered" when a polynucleotide has been transferred into the cell by any suitable means of artificial manipulation, or where the cell is a progeny of the originally altered cell that has inherited the polynucleotide. The polynucleotide will often comprise a transcribable sequence encoding a protein of interest, which enables the cell to express the protein at an elevated level. The genetic alteration is said to be "inheritable" if progeny of the altered cell have the same alteration.

The term "antibody" as used in this disclosure refers to both polyclonal and monoclonal antibody. The ambit of the term deliberately encompasses not only intact immunoglobulin molecules, but also such fragments and derivatives of immunoglobulin molecules (such as single chain Fv constructs, diabodies, and fusion constructs) as may be prepared by techniques known in the art, and retaining a desired antibody binding specificity.

### General Techniques

For further elaboration of general techniques described herein, the practitioner can refer to standard textbooks and reviews in cell biology, tissue culture, and embryology.

With respect to tissue culture and embryonic stem cells, the reader may wish to refer to Teratocarcinomas and embryonic stem cells: A practical approach (E.J. Robertson, ed., IRL Press Ltd. 1987); Guide to Techniques in Mouse Development (P.M. Wasserman et al. eds., Academic Press 1993); Embryonic Stem Cell Differentiation in Vitro (M.V. Wiles, Meth. Enzymol. 225:900, 1993); Properties and uses of Embryonic Stem Cells: Prospects for Application to Human Biology and Gene Therapy (P.D. Rathjen et al., Reprod. Fertil. Dev. 10:31, 1998).

General principles of preparation and culture of bone cells, and the repair of bone lesions can be found in Bone: The Osteoblast and Osteocyte (B.K. Hall, CRC Press 1990); Differentiation and Morphogenesis of Bone (B.K. Hall ed., CRC Press 1994); Principles of Bone Biology, (J.P. Bilezikian et al. eds., Academic Press 1996); and The Cellular and Molecular Basis of Bone Formation and Repair (V. Rosen & S. Thies, R.G. Landes Co. 1995). Other reading of interest includes The Bone People (K. Hulme, Viking Press 1986); and Bone Appetit (B.E. Romano, West Coast Media Group 1998).

Methods in molecular genetics and genetic engineering are described in Molecular Cloning: A Laboratory Manual, 2nd Ed. (Sambrook et al., 1989); Oligonucleotide Synthesis (M.J. Gait, ed., 1984); Animal Cell Culture (R.I. Freshney, ed., 1987); the series Methods in Enzymology (Academic Press); Gene Transfer Vectors for Mammalian Cells (J.M. Miller & M.P. Calos, eds., 1987); Current Protocols in Molecular Biology and Short Protocols in Molecular Biology, 3rd Edition (F.M. Ausubel et al., eds., 1987 & 1995); and Recombinant DNA Methodology II (R. Wu ed., Academic Press 1995). Reagents, cloning vectors, and kits for genetic manipulation referred to in this disclosure are available from commercial vendors such as BioRad, Stratagene, Invitrogen, and ClonTech.

### Sources of Stem Cells

### Embryonic Stem Cells

Embryonic stem cells have been isolated from blastocysts of members of the primate species (Thomson et al., Proc. Natl. Acad. Sci. USA 92:7844, 1995). Human embryonic stem (hES) cells can be prepared from human blastocyst cells using the techniques described by Thomson et al. (U.S. Patent 5,843,780; Science 282:1145, 1998; Curr. Top. Dev. Biol. 38:133 ff., 1998) and Reubinoff et al, Nature Biotech. 18:399,2000. Equivalent cell types to hES cells include their pluripotent derivatives, such as primitive ectoderm-like (EPL) cells, as outlined in WO 01/51610 (Bresagen).

Briefly, human blastocysts are obtained from human in vivo preimplantation embryos. Alternatively, in vitro fertilized (IVF) embryos can be used, or one-cell human embryos can be expanded to the blastocyst stage (Bongso et al., Hum Reprod 4: 706, 1989). Embryos are cultured to the blastocyst stage in G1.2 and G2.2 medium (Gardner et al., Fertil. Steril. 69:84, 1998). The zona pellucida is removed from developed blastocysts by brief exposure to pronase (Sigma). The inner cell masses are isolated by immunosurgery, in which blastocysts are exposed to a 1:50 dilution of rabbit anti-human spleen cell antiserum for 30 min, then washed for 5 min three times in DMEM, and exposed to a 1:5 dilution of Guinea pig complement (Gibco) for 3 min (Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers.

After 9 to 15 days, inner cell mass-derived outgrowths are dissociated into clumps, either by exposure to calcium and magnesium-free phosphate-buffered saline (PBS) with 1 mM EDTA, by exposure to dispase or trypsin, or by mechanical dissociation with a micropipette; and then replated on mEF in fresh medium. Growing colonies having undifferentiated morphology are individually selected by micropipette, mechanically dissociated into clumps, and replated. ES-like morphology is characterized as compact colonies with apparently high nucleus to cytoplasm ratio and prominent nucleoli. Resulting ES cells are then routinely split every 1-2 weeks by brief trypsinization, exposure to Dulbecco's PBS (containing 2 mM EDTA), exposure to type IV collagenase (~200 U/mL; Gibco) or by selection of individual colonies by micropipette. Clump sizes of about 50 to 100 cells are optimal.

### Embryonic Germ Cells

Human Embryonic Germ (hEG) cells can be prepared from primordial germ cells present in human fetal material taken about 8-11 weeks after the last menstrual period. Suitable preparation methods are described in Shamblott et al., Proc. Natl. Acad. Sci. USA 95:13726, 1998 and U.S. Patent 6,090,622.

Briefly, genital ridges are rinsed with isotonic buffer, then placed into 0.1mL 0.05% trypsin/0.53 mM sodium EDTA solution (BRL) and cut into <1 mm³ chunks. The tissue is then pipetted through a 100 µL tip to further disaggregate the cells. It is incubated at 37°C for ~5 min, then ~3.5 mL EG growth medium is added. EG growth medium is DMEM, 4500 mg/L D-glucose, 2200 mg/L mM NaHCO₃; 15% ES qualified fetal calf serum (BRL); 2 mM glutamine (BRL); 1 mM sodium pyruvate (BRL); 1000-2000 U/mL human recombinant leukemia inhibitory factor (LIF, Genzyme); 1-2 ng/mL human recombinant bFGF (Genzyme); and 10 µM forskolin (in 10% DMSO). In an alternative approach, EG cells are isolated using hyaluronidase%collagenase/DNAse. Gonadal anlagen or genital ridges with mesenteries are dissected from fetal material, the genital ridges are rinsed in PBS, then placed in 0.1 mL HCD digestion solution (0.01 % hyaluronidase type V, 0.002% DNAse I, 0.1% collagenase type IV, all from Sigma prepared in EG growth medium). Tissue is minced, incubated 1 h or overnight at 37°C, resuspended in 1-3 mL of EG growth medium, and plated onto a feeder layer.

Ninety-six well tissue culture plates are prepared with a sub-confluent layer of feeder cells (e.g., STO cells, ATCC No. CRL 1503) cultured for 3 days in modified EG growth medium free of LIF, bFGF or forskolin, inactivated with 5000 rad γ-irradiation. ~0.2 mL of primary germ cell (PGC) suspension is added to each of the wells. The first passage is done after 7-10 days in EG growth medium, transferring each well to one well of a 24-well culture dish previously prepared with irradiated STO mouse fibroblasts. The cells are cultured with daily replacement of medium until cell morphology consistent with EG cells is observed, typically after 7-30 days or 1-4 passages.

### Propagation of pPS Cells in an Undifferentiated State

pPS cells can be propagated continuously in culture, using culture conditions that promote proliferation without promoting differentiation. Exemplary serum-containing ES medium is made with 80% DMEM (such as Knock-Out DMEM, Gibco), 20% of either defined fetal bovine serum (FBS, Hyclone) or serum replacement (WO 98/30679), 1% non-essential amino acids, 1 mM L-glutamine, and 0.1 mM β-mercaptoethanol. Just before use, human bFGF is added to 4 ng/mL (WO 99/20741, Geron Corp.).

Conventionally, ES cells are cultured on a layer of feeder cells, typically fibroblasts derived from embryonic or fetal tissue. Embryos are harvested from a CF1 mouse at 13 days of pregnancy, transferred to 2 mL trypsin/EDTA, finely minced, and incubated 5 min at 37°C. 10% FBS is added, debris is allowed to settle, and the cells are propagated in 90% DMEM , 10% FBS, and 2 mM glutamine. To prepare a feeder cell layer, cells are irradiated to inhibit proliferation but permit synthesis of factors that support ES cells (~4000 rads γ-irradiation). Culture plates are coated with 0.5% gelatin overnight, plated with 375,000 irradiated mEFs per well, and used 5 h to 4 days after plating. The medium is replaced with fresh hES medium just before seeding pPS cells.

Scientists at Geron have discovered that pPS cells can alternatively be maintained in an undifferentiated state even without feeder cells (WO 01/51616). The environment for feeder-free cultures includes a suitable culture substrate, particularly an extracellular matrix such as Matrigel® or laminin. The pPS cells are plated at >15,000 cells cm⁻² (optimally 90,000 cm⁻² to 170,000 cm⁻²). Typically, enzymatic digestion is halted before cells become completely dispersed (say, ~5 to 20 min with collagenase IV). Clumps of ~10-2000 cells are then plated directly onto the substrate without further dispersal.

Feeder-free cultures are supported by a nutrient medium typically conditioned by culturing irradiated primary mouse embryonic fibroblasts, telomerized mouse fibroblasts, or fibroblast-like cells derived from pPS cells. Medium can be conditioned by plating the feeders at a density of ~5-6 × 10⁴ cm⁻² in a serum free medium such as KO DMEM supplemented with 20% serum replacement and 4 ng/mL bFGF. Medium that has been conditioned for 24 h is filtered through a 0.2 µm membrane, supplemented with a further ~8 ng/mL bFGF, and used to support pPS cell culture for 1-2 days.

Under the microscope, ES cells appear with high nuclear/cytoplasmic ratios, prominent nucleoli, and compact colony formation with poorly discernable cell junctions. Primate ES cells may express one or more of the stage-specific embryonic antigens (SSEA) 3 and 4, and markers detectable using antibodies designated Tra-1-60 and Tra-1-81 (Thomson et al., Science 282:1145, 1998). Undifferentiated hES cells also typically express Oct-4 and TERT, as detected by RT-PCR. Differentiation of hES cells in vitro typically results in the loss of these markers (if present) and increased expression of SSEA-1.

### Materials and procedures for prepaying mesenchymal cells and osteoblasts

A culture medium or growth environment of this invention may be used for culturing, differentiating, or reprogramming human embryonic stem cells that enriches for cells with the desired phenotype (either by outgrowth of the desired cells, or by inhibition or killing of other cell types).

Differentiation can optionally be initiated by formation of embryoid bodies or aggregates: for example, by overgrowth of a donor pPS cell culture, or by culturing pPS cells in suspension in culture vessels having a substrate with low adhesion properties which allows EB formation. pPS cells are harvested by brief collagenase digestion, dissociated into clusters, and plated in non-adherent cell culture plates. The aggregates are fed every few days, and then harvested after a suitable period, typically 4-8 days. Alternatively or in addition, the differentiation process can be initiated by culturing in a non-specific differentiation paradigm: for example, by including retinoic acid (RA) or dimethyl sulfoxide (DMSO) in the culture medium; or by withdrawing from the usual extracellular matrix upon which the cells are cultured. See U.S. patent application 60/213,740 and International patent publication WO 01/51616.

Production of relatively homogeneous populations of mesenchymal cells, particularly of the osteoblast lineage can be achieved by culturing pPS cells (either undifferentiated, or after differentiation has been initiated) in a growth environment containing factors beneficial to such cells, such as one or more of the following:
- Bone morphogenic proteins, exemplified by BMP-2, BMP-3, BMP-4, BMP-6 and BMP-7
- TGF-β, exemplified by TGF-β1, TGF-β2, and TGF-β3 and their analogs, and other members of the TGF-β superfamily that bind a TGF-β receptor
- Ligands for the Vitamin D receptor. Exemplary is 1,25-dihydroxy Vitamin D3. Other analogs are known (see, for example, Tsugawa et al., Biol. Pharm. Bull. 23:66, 2000)
It is recognized that specific antibody to the receptors of any of these factors are functionally equivalent ligands that can be used in place of (or in addition to) the factors listed. Other additives that may be used included:
- other morphogens, such as a fibroblast growth factor like basic FGF
- a glucocorticoid
- dexamethasone, or other small-molecule osteoblast maturation factor
- ascorbic acid (or an analog thereof, such as ascorbic acid-2-phosphate), which is a cofactor for proline hydroxylation that occurs during the course of collagen synthesis
- β-glycerophosphate, or other substrate for alkaline phosphatase during the process of mineralization
- a source of calcium (may or may not already be present in sufficient concentration the basal medium)
The cells can also supported on a substrate coated with an appropriate material conducive to growth of the desired cell phenotype, or cultured in a medium containing the components of such a material. Matrigel®, laminin, collagen (especially collagen type I), glycosaminoglycans, osteocalcin, and osteonectin may all be suitable as an extracellular matrix, by themselves or in various combinations. Also suitable for growing osteoblast lineage cells are gel-derived glasses, silica gels, and sol-gel-derived titania (Saravanapavan et al., J. Biomed Mater. Res. 54:608, 2001; Dieudonne et al., Biomaterials 34:3041, 2002).

The cells can be characterized according to a number of phenotypic criteria. Relatively undifferentiated mesenchymal cells can be recognized by their characteristic mononuclear ovoid, stellate shape or spindle shape, with a round to oval nucleus and a poorly defined cell border. The oval elongate nuclei typically have prominent nucleoli and a mix of hetero- and euchromatin. These cells have little cytoplasm but many thin processes that appear to extend from the nucleus. They will typically stain for one, two, three or more of the following markers: CD106 (VCAM), CD166 (ALCAM), CD29, CD44, GATA-4, and alkaline phosphatase, while being negative for hematopoietic lineage cell markers (CD14 or CD45). Mesenchymal stem cells may also express STRO-1.

Under appropriate conditions, early mesenchymal cells can differentiate further into many adult connective tissue cell types, such as fibroblasts, chondroblasts, osteoblasts, odontoblasts, reticular cells or adipocytes. Accordingly, mesenchymal stem cells can be identified by their capacity to form progeny of one or more specialized mesenchymal lineages.

Osteoblasts and bone precursor cells will typically have at least one characteristic (typically at least three or five characteristics) from the following list:
- density between ~1.050 and ~1.090 g cm⁻³
- positive for osteonectin (positive in osteoblasts and precursors)
- positive for osteocalcin (specific for mature osteoblasts)
- a cell diameter of ~8 to ~70 µm
- cuboidal shape
- upregulated production alkaline phosphatase, especially in response to presence of BMP
- positive for type I collagen (procollagen) or for vimentin
- positive for other osteoblast-specific markers, such as BMP receptors, PTH receptors, or CD105 (endoglin)
- evidence of ability to mineralize the external surroundings, or synthesize calcium-containing extracellular matrix
The skilled reader will know that chondrocytes typically express Type II collagen, aggrecan, or proteoglycans that stain with alcian blue. In the mature form, chondrocytes will be less than 1 % positive for elastin, Type I collagen, Type X collagen, or osteocalcin. Hematopoietic cell populations and their precursors will bear such markers as re CD45, CD34, CD13, AC133, hemoglobin, surface antibody, and Class It histocompatibility antigens. Under appropriate circumstances, replicative hematopoietic cells will form colonies in an assay for hematopoietic colony forming units (CFU). Cardiomyocytes and their precursors typically express cardiac troponin I (cTnl), cardiac troponin T (cTnT), atrial natriuretic factor (ANF), and alpha cardiac myosin heavy chain (MHC). Fibroblasts have readily identifiable morphology and typically express collagenase I, and tissue inhibitor of metalloproteinase I (TIMP-1). Striated muscle cells typically express contractile proteins such as skeletal α-actin, skeletal myosin heavy and light chains, and tropomyosin. Earlier myogenic markers are myoD and myogenin. Tendon and ligament tissue stains for type I collagen in a unidirectional fiber arrangement. Early tendon and chondrocyte progenitors typically express scleraxis. Adipocytes typically stain with oil red O showing lipid accumulation, and express peroxisome proliferation-activated receptor γ2 (PPARγ2), lipoprotein lipase (LPL), and fatty acid binding protein (aP2).

Tissue-specific markers can be detected using any suitable immunological technique such as flow immunocytochemistry or affinity adsorption for cell-surface markers, immunocytochemistry (for example, of fixed cells or tissue sections) for intracellular or cell-surface markers, Western blot analysis of cellular extracts, and enzyme-linked immunoassay, for cellular extracts or products secreted into the medium. Expression of an antigen by a cell is said to be "antibody-detectable" if a significantly detectable amount of antibody will bind to the antigen in a standard immunocytochemistry or flow cytometry assay, optionally after fixation of the cells, and optionally using a labeled secondary antibody or other conjugate (such as a biotin-avidin conjugate) to amplify labeling.

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. See U.S. Patent 5,843,780 for details of general technique, and International Patent Publication WO 99/39724 for osteoblast-specific PCR primers. Sequence data for other markers listed in this disclosure can be obtained from public databases such as GenBank (URL www.ncbi.nlm.nih.gov:80/entrez). Expression at the mRNA level is said to be "detectable" according to one of the assays described in this disclosure if the performance of the assay on cell samples according to standard procedures in a typical controlled experiment results in clearly discernable hybridization or amplification product. Expression of tissue-specific markers as detected at the protein or mRNA level is considered positive if the level is at least 2-fold, and preferably more than 10- or 50-fold above that of a control cell, such as an undifferentiated pPS cell or other unrelated cell type.

The presence of alkaline phosphatase activity can be detected by fixing the cells with 4% paraformaldehyde, and then developing with Vector Red as a substrate, as described by the manufacturer (Vector Laboratories, Burlingame CA). Calcium accumulation inside cells and deposition into matrix proteins can be measured by culturing in ⁴⁵Ca⁺⁺, washing and reculturing, and then determining any radioactivity present inside the cell or deposited into the extracellular matrix (U.S. Patent 5,972,703); or by assaying culture substrate for mineralization using a Ca⁺⁺ assay kit (Sigma Kit #587).

Once markers have been identified on the surface of cells of the desired phenotype, they can be used for immunoselection to further enrich the population by techniques such as immunopanning or antibody-mediated fluorescence-activated cell sorting.

### Genetic alteration of differentiated cells

It may be desirable that the cells have the ability to replicate in certain drug screening and therapeutic applications, and to provide a reservoir for the generation of mesenchymal cells and osteoblasts. The cells of this invention can optionally be telomerized to increase their replication potential, either before or after they progress to restricted developmental lineage cells or terminally differentiated cells. pPS cells that are telomerized may be taken down the differentiation pathway described earlier; or differentiated cells can be telomerized directly.

Cells are telomerized by genetically altering them by transfection or transduction with a suitable vector, homologous recombination, or other appropriate technique, so that they express the telomerase catalytic component (TERT), typically under a heterologous promoter that increases telomerase expression beyond what occurs under the endogenous promoter. Particularly suitable is the catalytic component of human telomerase (hTERT), provided in International Patent Application WO 98/14592. For certain applications, species homologs like mouse TERT (WO 99/27113) can also be used. Transfection and expression of telomerase in human cells is described in Bodnar et al., Science 279:349, 1998 and Jiang et al., Nat. Genet. 21:111, 1999. In another example, hTERT clones (WO 98/14592) are used as a source of hTERT encoding sequence, and spliced into an EcoRI site of a PBBS212 vector under control of the MPSV promoter, or into the EcoRI site of commercially available pBABE retrovirus vector, under control of the LTR promoter.

Differentiated or undifferentiated pPS cells are genetically altered using vector containing supernatants over a 8-16 h period, and then exchanged into growth medium for 1-2 days. Genetically altered cells are selected using 0.5-2.5 µg/mL puromycin, and recultured. They can then be assessed for hTERT expression by RT-PCR, telomerase activity (TRAP assay), immunocytochemical staining for hTERT, or replicative capacity. The following assay kits are available commercially for research purposes: TRAPeze® XL Telomerase Detection Kit (Cat. s7707; Intergen Co., Purchase NY); and TeIoTAGGG Telomerase PCR ELISAplus (Cat. 2,013,89; Roche Diagnostics, Indianapolis IN). TERT expression can also be evaluated at the mRNA by RT-PCR. Available commercially for research purposes is the LightCycler TeIoTAGGG hTERT quantification kit (Cat. 3,012,344; Roche Diagnostics). Continuously replicating colonies will be enriched by further culturing under conditions that support proliferation, and cells with desirable phenotypes can optionally be cloned by limiting dilution.

pPS cells can be differentiated into multipotent or committed mesenchymal cells, and then genetically altered to express TERT.

pPS cells can be genetically altered to express TERT, and then differentiated into osteoblast precursors or terminally differentiated cells. Successful modification to increase TERT expression can be determined by TRAP assay, or by determining whether the replicative capacity of the cells has improved.

Depending on the application, other methods of immortalization may also be used, such as transforming the cells with DNA encoding myc, the SV40 large T antigen, or MOT-2 (U.S. Patent 5,869,243, International Patent Applications WO 97/32972 and WO 01/23555). Transfection with oncogenes or oncovirus products is less suitable when the cells are to be used for therapeutic purposes. Telomerized cells are of particular interest where it is advantageous to have cells that can proliferate and maintain their karyotype - for example, in pharmaceutical screening, and in therapeutic protocols where differentiated cells are administered to an individual in order to augment musculoskeletal function.

The cells can also be genetically altered in order to enhance their ability to be involved in tissue regeneration, or to deliver a therapeutic gene to a site of administration. A vector is designed using the known encoding sequence for the desired gene, operatively linked to a promoter that is either pan-specific or specifically active in the differentiated cell type. Of particular interest are cells that are genetically altered to express a bone morphogenic protein, such as BMP-2 or BMP-4. See WO 99/39724. Production of these or other growth factors at the site of administration may enhance the beneficial effect of the administered cell, or increase proliferation or activity of host cells neighboring the treatment site.

### Use of mesenchymal stem cells, osteoblast precursors and terminally differentiated cells

These cell populations can be used for a number of important research, development, and commercial purposes.

The cells can be used to prepare a cDNA library relatively uncontaminated with cDNA preferentially expressed in cells from other lineages. For example, mesenchymal progenitor cells or osteoblasts are collected by centrifugation at 1000 rpm for 5 min, and then mRNA is prepared from the pellet by standard techniques (Sambrook et al., supra). After reverse transcribing into cDNA, the preparation can be subtracted with cDNA from undifferentiated pPS cells, other progenitor cells, or end-stage cells from the osteoblast or any other developmental pathway.

The differentiated cells can also be used to prepare antibodies that are specific for markers of mesenchymal cells, osteoblasts, and intermediate precursors. Polyclonal antibodies can be prepared by injecting a vertebrate animal with cells of this invention in an immunogenic form. Production of monoclonal antibodies is described in such standard references as U.S. Patents 4,491,632, 4,472,500 and 4,444,887, and Methods in Enzymology 73B:3 (1981). Specific antibody molecules can also be produced by contacting a library of immunocompetent cells or viral particles with the target antigen, and growing out positively selected clones. See Marks et al., New Eng. J. Med. 335:730, 1996, and McGuiness et al., Nature Biotechnol. 14:1449, 1996. A further alternative is reassembly of random DNA fragments into antibody encoding regions, as described in EP patent application 1,094,108 A.

By positively selecting using pPS and negatively selecting using cells bearing more broadly distributed antigens (such as differentiated embryonic cells) or adult-derived stem cells, the desired specificity can be obtained. The antibodies in turn can be used to identify or rescue mesenchymal cells of a desired phenotype from a mixed cell population, for purposes such as costaining during immunodiagnosis using tissue samples, and isolating precursor cells from terminally differentiated osteoblasts and cells of other lineages.

The cells are also of interest in identifying expression patterns of transcripts and newly synthesized proteins that are characteristic for mesenchymal cells, and may assist in directing the differentiation pathway or facilitating interaction between cells. Expression patterns of the differentiated cells are obtained and compared with control cell lines, such as undifferentiated pPS cells, other types of committed precursor cells (such as pPS cells differentiated towards other lineages), or terminally differentiated cells.

The use of microarray in analyzing gene expression is reviewed generally by Fritz et al Science 288:316, 2000; "Microarray Biochip Technology", L Shi, www.Gene-Chips.com. An exemplary method is conducted using a Genetic Microsystems array generator, and an Axon GenePix™ Scanner. Microarrays are prepared by first amplifying cDNA fragments encoding marker sequences to be analyzed, and spotted directly onto glass slides To compare mRNA preparations from two cells of interest, one preparation is converted into Cy3-labeled cDNA, while the other is converted into Cy5-labeled cDNA. The two cDNA preparations are hybridized simultaneously to the microarray slide, and then washed to eliminate non-specific binding. The slide is then scanned at wavelengths appropriate for each of the labels, the resulting fluorescence is quantified, and the results are formatted to give an indication of the relative abundance of mRNA for each marker on the array.

### Drug screening

Mesenchymal cells and osteoblasts can be used to screen for factors (such as solvents, small molecule drugs, peptides, oligonucleotides) or environmental conditions (such as culture environment or manipulation) that affect the characteristics of such cells and their various progeny.

In some applications, pPS cells (undifferentiated or differentiated) are used to screen factors that promote maturation into later-stage mesenchymal precursors, or terminally differentiated cells, or to promote proliferation and maintenance of such cells in long-term culture. For example, candidate maturation factors or growth factors are tested by adding them to cells in different wells, and then determining any phenotypic change that results, according to desirable criteria for further culture and use of the cells. In one illustration, pPS derived cells with an early mesenchymal phenotype are used to screen factors for their ability to direct differentiation towards particular cell types, such as myocytes, cartilage, or adipocytes.

Other screening applications relate to the testing of pharmaceutical compounds for their effect on musculoskeletal tissue maintenance or repair. In one illustration, pPS derived cells with osteoblast characteristics are used to screen factors for their ability to affect calcium deposition. Screening may be done either because the compound is designed to have a pharmacological effect on the cells, or because a compound designed to have effects elsewhere may have unintended side effects on cells of this tissue type. The screening can be conducted using any of the precursor cells or terminally differentiated cells.

The reader is referred generally to the standard textbook "In vitro Methods in Pharmaceutical Research", Academic Press, 1997, and U.S. Patent 5,030,015. Assessment of the activity of candidate pharmaceutical compounds generally involves combining the differentiated cells of this invention with the candidate compound, either alone or in combination with other drugs. The investigator determines any change in the morphology, marker phenotype, or functional activity of the cells that is attributable to the compound (compared with untreated cells or cells treated with an inert compound), and then correlates the effect of the compound with the observed change.

Cytotoxicity can be determined in the first instance by the effect on cell viability, survival, morphology, and the expression of certain markers and receptors. Effects of a drug on chromosomal DNA can be determined by measuring DNA synthesis or repair. [³H]-thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to A. Vickers (pp 375-410 in "In vitro Methods in Pharmaceutical Research," Academic Press, 1997) for further elaboration.

Effect of cell function can be assessed using any standard assay to observe phenotype or activity of mesenchymal cells or osteoblastoid cells, such as receptor binding, matrix deposition, or calcium processing - either in cell culture or in an appropriate animal model.

### Therapeutic use

To determine the suitability of cell compositions for therapeutic administration, the cells can first be tested in a suitable animal model. At one level, cells are assessed for their ability to survive and maintain their phenotype in vivo. Cell compositions are administered to immunodeficient animals (such as nude mice, or animals rendered immunodeficient chemically or by irradiation). Tissues are harvested after a period of regrowth, and assessed as to whether pPS derived cells are still present.

This can be performed by administering cells that express a detectable label (such as green fluorescent protein, or β-galactosidase); that have been prelabeled (for example, with BrdU or [³H]thymidine), or by subsequent detection of a constitutive cell marker (for example, using human-specific antibody). The presence and phenotype of the administered cells can be assessed by immunohistochemistry or ELISA using human-specific antibody, or by RT-PCR analysis using primers and hybridization conditions that cause amplification to be specific for human polynucleotides, according to published sequence data.

Suitability can also be determined by assessing the degree of recuperation that ensues from treatment with a cell population of mesenchymal cells. For example, the regenerative capacity for bone and cartilage can be determined using a rat calvarial defect model (U.S. Patent 6,200,606). There are established animal models for treatment of mandibular defects, maxillary alveolar clefts, and ostectomy gaps in rabbits, dogs, and monkeys (WO 99/39724). Deposition of bone into model lesions can be monitored by X-ray analysis and other techniques. Reconstituted bony tissue can be evaluated for function using standard biomechanical testing. See Minamide et al., Spine 24:1863, 1999; Takahashi et al., J. Neurosurg. 90 (4 suppl.):224, 1999; Helm et al., J. Neurosurg. 88:354, 1997.

After adequate testing, differentiated cells can be used for tissue reconstitution or regeneration in a human patient or other subject in need of such treatment. The cells are administered in a manner that permits them to graft or migrate to the intended tissue site and reconstitute or regenerate the functionally deficient area. Medical indications for such treatment include regeneration of musculoskeletal defects, fracture repair, spinal chord rehabilitation, installation of prosthetics, and repair of osteoporosis-related injury.

Administration of the composition will depend on the musculoskeletal site being repaired. For example, osteogenesis can be facilitated in concordance with a surgical procedure remodel tissue or insert a split, or a prosthetic device such as a hip replacement. In other circumstances, invasive surgery will not be required, and the composition can be administered by injection or (for repair of the vertebral column) using a guidable endoscope.

The mesenchymal cells and osteoblasts can be supplied in the form of a pharmaceutical composition, comprising an isotonic excipient prepared under sufficiently sterile conditions for human administration. For general principles in medicinal formulation, the reader is referred to Cell Therapy: Stem Cell Transplantation, Gene Therapy, and Cellular Immunotherapy, by G. Morstyn & W. Sheridan eds, Cambridge University Press, 1996; and Hematopoietic Stem Cell Therapy, E.D. Ball, J. Lister & P. Law, Churchill Livingstone, 2000. Choice of the cellular excipient and any accompanying elements of the composition will be adapted in accordance with the device used for administration.

If desired, the cell preparation can further include or be coadministered with a complementary bioactive factor such as a synthetic glucocorticoid like dexamethasone, or a bone morphogenic protein, such as BMP-2 or BMP-4. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a granular ceramic, or a biopolymer such as gelatin, collagen, osteonectin, fibrinogen, or osteocalcin. Porous matrices can be synthesized according to standard techniques (e.g., Mikos et al., Biomaterials 14:323, 1993; Mikos et al., Polymer 35:1068, 1994; Cook et al., J. Biomed. Mater. Res. 35:513, 1997).

The composition may optionally be packaged in a suitable container with written instructions for a desired purpose, such as the reconstitution of mesenchymal cell function to improve some musculoskeletal abnormality.

### REFERENCE EXAMPLES

### Reference Example 1: Feeder-free propagation of embryonic stem cells

Established lines of undifferentiated human embryonic stem (hES) cells were maintained in a culture environment essentially free of feeder cells.

Feeder-free cultures were maintained using conditioned medium prepared using primary mouse embryonic fibroblasts isolated according to standard procedures (WO 01/51616). Fibroblasts were harvested from T150 flasks by washing once with Ca⁺⁺/Mg⁺⁺ free PBS and incubating in 1.5-2 mL trypsin/EDTA (Gibco) for ~5 min. After the fibroblasts detached from the flask, they were collected in mEF media (DMEM + 10% FBS). The cells were irradiated at 4000 rad, counted and seeded at ~55,000 cells cm⁻² in mEF media (525,000 cells/well of a 6 well plate).

After at least 4 h, the medium were exchanged with SR containing ES medium (80% knockout DMEM (Gibco BRL, Rockville MD), 20% knockout serum replacement (Gibco ), 1% Non-essential amino acids (Gibco), 1 mM L-glutamine (Gibco), 0.1 mM β-mercaptoethanol (Sigma, St. Louis, MO), supplemented with 4 ng/mL recombinant human basic fibroblast growth factor (bFGF; Gibco). About 0.3-0.4 mL of medium were conditioned per cm² of plate surface area. Before addition to the hES cultures, the conditioned medium was supplemented with 4 ng/mL of human bFGF.

Plates for culturing the hES cells were coated with Matrigel® (Becton-Dickinson, Bedford MA) by diluting stock solution ~1:30 in cold KO DMEM, dispensing at 0.75-1.0 mL per 9.6 cm² well, and incubating for 4 h at room temp or overnight at 4°C.

hES cultures were passaged by incubation in ~200 U/mL collagenase IV for about 5'-10 minutes at 37°C. Cells were harvested by removing individual colonies up with a Pipetman™ under a microscope or scraping, followed by gentle dissociation into small clusters in conditioned medium, and then seeded onto Matrigel® coated plates. About one week after seeding the cultures became confluent and could be passaged. Cultures maintained under these conditions for over 180 days continued to display ES-like morphology.

Immunocytochemistry was performed by incubating sample wells with primary antibody for SSEA-4 (1:20), Tra-1-60 (1:40) and Tra-1-81 (1:80), diluted in knockout DMEM at 37°C for 30 min. The cells were washed with warm knockout DMEM and fixed in 2% paraformaldehyde for 15 min, and then with PBS. The cells were incubated with 5% goat serum in PBS at room temp for 30 min, followed by the FITC-conjugated goat anti-mouse IgG (1: 125) (Sigma) for 30 min. Cells were washed, stained with DAPI and mounted.

Cells were also examined for expression of alkaline phosphatase, a marker for undifferentiated ES cells. This was performed by culturing the cells on chamber slides, fixing with 4 % paraformaldehyde for 15 min, and then washing with PBS. Cells were then incubated with alkaline phosphatase substrate (Vector Laboratories, Inc., Burlingame, CA) at room temperature in the dark for 1 h. Slides were rinsed for 2-5 min in 100% ethanol before mounting.

**Figure 1** shows marker expression on the hES cells detected by histochemistry. SSEA-4, Tra-1-60, Tra-1-81, and alkaline phosphatase were expressed by the hES colonies, as seen for the cells on feeders - but not by the differentiated cells in between the colonies.

Expression of the undifferentiated hES cell markers was assayed by reverse-transcriptase PCR amplification. For radioactive relative quantification of individual gene products, QuantumRNA™ Alternate18S Internal Standard primers (Ambion, Austin TX, USA) were employed according to the manufacturer's instructions. Briefly, the linear range of amplification of a particular primer pair was determined, then coamplified with the appropriate mixture of alternate18S primers:competimers to yield PCR products with coinciding linear ranges. Before addition of AmpliTaq™ (Roche) to PCR reactions, the enzyme was pre-incubated with the TaqStart™ antibody (ProMega) according to manufacturer's instructions. Radioactive PCR reactions were analyzed on 5% non-denaturing polyacrylamide gels, dried, and exposed to phosphoimage screens (Molecular Dynamics) for 1 hour. Screens were scanned with a Molecular Dynamics Storm 860 and band intensities were quantified using ImageQuant™ software. Results are expressed as the ratio of radioactivity incorporated into the hTERT or Oct-4 band, standardized to the radioactivity incorporated into the 18s band. Primer sequences used in this experiment can be found in PCT publication WO 01/51616.

The transcription factor Oct-4 is normally expressed in the undifferentiated hES cells and is down-regulated upon differentiation. Cells maintained on Matrigel® in conditioned medium for 21 days expressed hTERT and Oct-4. Telomerase activity was measured by TRAP assay (Kim et al., Science 266:2011, 1997; Weinrich et al., Nature Genetics 17:498, 1997). Cells maintained in the feeder-free culture environment showed positive telomerase activity after over 40 days in culture.

Pluripotency of the undifferentiated cells cultured without feeders was determined by forming embryoid bodies in suspension culture for 4 days, and then culturing on poly-ornithine coated plates for 7 days. Immunocytochemistry showed staining patterns consistent with cells of the neuron and cardiomyocyte lineages, and cells staining for α-fetoprotein, a marker of endoderm lineage. The undifferentiated cells were also tested for their ability to form teratomas by intramuscular injection into SCID mice. Resulting tumors were excised after 78-84 days. Cell types from all three germ layers were identified by histological analysis.

### Reference Example 2: Establishment of a differentiated cell line

Embryoid bodies were produced as follows. Confluent monolayer cultures of hES cells were harvested by incubating in 1 mg/mL collagenase for 5-20 min, and the cells were scraped from the plate. The cells were then dissociated into clusters and plated in non-adherent cell culture plates (Costar) in a medium composed of 80% KO ("knockout") DMEM (Gibco) and 20% non-heat-inactivated FBS (Hyclone), supplemented with 1% non-essential amino acids, 1 mM glutamine, 0.1 mM β-mercaptoethanol. The cells were seeded at a 1:1 or 1:2 ratio in 2 mL medium per well (6 well plate). The EBs were fed every other day by the addition of 2 mL of medium per well. When the volume of medium exceeded 4 mL/well, the EBs were collected and resuspended in fresh medium. After 4-8 days in suspension, the EBs were plated onto a substrate.

A differentiated cell line was established by harvesting the embryoid body derived cells and allowing them to differentiated further. The cells were harvested by incubating in 2 mg/mL Collagenase type II in PBS for 30 min at 37°C. The cells were dissociated, centrifuged, resuspended in differentiation medium, and plated in a 6-well plate. The proliferating cells were passaged in hEF medium (90% DMEM, 10% heat-inactivated FBS, 0.1 mM non-essential amino acids, and 2 mM L-glutamine), and fed every 2-3 days. After two passages, the cell population appeared homogeneous with morphological characteristics of fibroblasts. This cell line was designated **HEF1.**

A subpopulation was transduced for expression of human telomerase reverse transcriptase (hTERT). This was accomplished by infecting with a retroviral construct pBABE *puro* hTERT, containing the hTERT coding sequence driven by the MoLV LTR and the puromycin-resistance gene driven by the SV40 early promoter. Growth medium was replaced with a mixture containing 5 mL of retroviral stock (1 x 10⁶ pfu/mL) and 4 µg/mL polybrene, and incubating at 37°C. After 8 h, an additional 5 mL of the retrovirus/polybrene mixture was added and the cells were incubated at 37°C. On the next day, the retrovirus/polybrene mixture was removed and replaced with fresh growth medium. The next day, the medium was replaced with growth medium supplemented with 0.5 micrograms/mL puromycin. Cells were split about once a week at a ratio of 1:4 for 8 weeks in puromycin-containing medium, and then tested for telomerase activity.

**Figure 2** (Panel A) shows the morphology of the telomerized HEF1 cell line. Panel B (below) shows telomerase activity, as measured in the TRAP assay. Cells transduced with the hTERT expression cassette showed positive telomerase activity at 20 or 65 days after transduction. The untransduced cell line, or cells transduced with the vector control showed no telomerase activity. Both the hTERT-transduced HEF1 cells, and cells transduced with vector control, doubled about once every 2 days, until the 38 day point, when the control cells stopped dividing. The hTERT-transfected cells continued proliferating beyond the 60 day point (30 doublings) at a consistent growth rate.

ES cell growth medium was conditioned as in Example 8, using HEF1 cells irradiated at 6000 rad, and seeded at ~4.1 to 5.5 x 10⁴ cells cm⁻². The medium was tested for its ability to support growth of the H9 hES cell line cultured on a Matrigel® substrate. The hES cells have been maintained using the HEF1 conditioned medium for more than 4 passages, displaying morphology of undifferentiated ES cells, and maintaining expression of hTERT and Oct-4.

### Reference Example 3: Further differentiation to osteoblast-like cells

Human ES cells (H1 cell line, passage 30) were maintained in feeder-free conditions, as described earlier. For use in this experiment, hES cells were seeded at a density of ~1 × 10⁵ cm⁻² on Matrigel® in mEF conditioned medium. Telomerized HEF1 cells were plated at 3.1 × 10³ cm⁻² in 10% FBS, 1% non-essential amino acids and 2 mM L-glutamine in DMEM. Normal human mesenchymal stem cells (hMSC) were obtained from BioWhittaker Inc., MD (a subsidiary of Cambrex Co.). They were maintained in MSC growth medium (BioWhittaker Part #PT-3001) according to manufacturer's directions. The BJ5ta fibroblast cell line (Bodnar et al., Science 279:349, 1998) was maintained in a standard medium made from 10% FBS in 1:3 M199/DMEM.

Two days after the last passage, each culture medium was replaced with osteoblast induction medium (OIM) to induce differentiation. The OIM was based on MSC growth medium (ClonTech Cat. #PT-3238) (US Patent 5,486,359) supplemented with 0.1 µM dexamethasone, 5 µM ascorbic acid-2-phosphate, 10 mM β-glycerophosphate, and 100 ng/mL BMP-4. Cells were fed fresh OIM every 2-3 days.

After 11 days in OIM, all cells showed changes in cell morphology. HEF1 cells, hMSC and BJ cells changed from spindle to cuboidal shaped, and some cells became flatter. hES cells showed a heterogeneous morphology that appeared to be a mixed differentiated population.

Cells were fixed in 2% paraformaldehyde in PBS for 20 min, washed with PBS, and analyzed for osteoblast markers. Alkaline phosphatase (AP) was detected with Vector substrate (Vector Laboratories, Inc., Burlingame, CA). Expression of AP was clearly localized to clusters of cells differentiated H1 cells as well as HEF1, BJ and hMSC cells.

Matrix proteins produced by osteoblasts, collagen-1 and osteocalcin, were detected by immunostaining. Cultures were permeabilized by treatment with 100% EtOH for 2 min. After washing in PBS, cultures were incubated with 5% normal goat serum in PBS for 2 h, and then with primary rabbit antibody against collagen-1 (1:10, Monosan Cat. #P5041) or osteocalcin (1:50, Biomedical Technologies Inc. Cat. #13T593). Staining was developed with the FITC-labeled secondary goat anti-rabbit immunoglobulin (1:100, Southern Biotechnology Associates inc. Cat. #4050-02).

**Figure 3** shows the results. Panels A and B show immunocytochemistry for the markers osteocalcin and collagen-1. Panel C shows staining for alkaline phosphatase activity. These features are characteristic of cells of the osteoblast lineage.

These data are consistent with the hypothesis that both hES cells and HEF1 cells have the capacity to generate osteoblasts when subjected to an appropriate differentiation protocol in vitro.

## Claims

1. A culture medium for obtaining osteoprogenitors and/or osteoblasts from human embryonic stem (hES) cells or their progeny, comprising BMP-4, dexamethasone, and ascorbic acid or an analog thereof.

2. The culture medium of claim 1, further comprising beta-glycerophosphate.

3. A growth environment comprising a culture medium according to claim 1 or claim 2 and a supporting structure.

## Patentansprüche

1. Kulturmedium zum Erhalten von Osteoprogenitorzellen und/oder Osteoblasten aus menschlichen embryonalen Stammzellen (hES) oder deren Nachkommen, das BMP-4, Dexamethason und Ascorbinsäure oder ein Analog davon umfasst.

2. Kulturmedium nach Anspruch 1, das ferner Beta-Glycerophosphat aufweist.

3. Wachstumsumgebung, die ein Kulturmedium nach Anspruch 1 oder 2 und eine Stützstruktur aufweist.

## Revendications

1. Milieu de culture pour obtenir des ostéoprogéniteurs et/ou des ostéoblastes provenant de cellules souches embryonnaires humaines (hES) ou leur descendance, comprenant du BMP-4, de la dexaméthasone et de l'acide ascorbique ou un analogue de celui-ci.

2. Milieu de culture selon la revendication 1, comprenant en outre du bêta-glycérophosphate.

3. Environnement de croissance comprenant un milieu de culture selon la revendication 1 ou la revendication 2 et une structure de support.
